(19) Europäisches Patentamt
European Patent Office
Office européen des brevets

(11) **EP 3 311 137 B1**

(12) **EUROPEAN PATENT SPECIFICATION**

(45) Date of publication and mention
of the grant of the patent:
**24.07.2019 Bulletin 2019/30**

(51) Int Cl.:
***G01N 21/27*** *(2006.01)* ***G01N 21/79*** *(2006.01)*
***C07C 2/60*** *(2006.01)*

(21) Application number: **16731558.9**

(22) Date of filing: **16.06.2016**

(86) International application number:
**PCT/EP2016/063832**

(87) International publication number:
**WO 2016/202901 (22.12.2016 Gazette 2016/51)**

(54) **PROCESS FOR MONITORING THE CATALYTIC ACTIVITY OF AN IONIC LIQUID**

PROZESS ZUR ÜBERWACHUNG DER KATALYTISCHEN AKTIVITÄT EINER IONISCHEN FLÜSSIGKEIT

PROCÉDÉ POUR SURVEILLER L'ACTIVITÉ CATALYTIQUE D'UN LIQUIDE IONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2015 PCT/CN2015/081771**

(43) Date of publication of application:
**25.04.2018 Bulletin 2018/17**

(73) Proprietor: **Shell Internationale Research Maatschappij B.V.**
**2596 HR The Hague (NL)**

(72) Inventors:
• **LIU, Zhichang**
**Beijing 102249 (CN)**
• **ZHANG, Rui**
**Beijing 102249 (CN)**
• **ZHANG, Xuan**
**Beijing 102249 (CN)**
• **MENG, Xianghai**
**Beijing 102249 (CN)**
• **LIU, Haiyan**
**Beijing 102249 (CN)**
• **XU, Chunming**
**Beijing 102249 (CN)**
• **KLUSENER, Peter, Anton, August**
**1031 HW Amsterdam (NL)**

(74) Representative: **Shell Legal Services IP**
**p/a Carel van Bylandtlaan 16**
**2596 HR Den Haag (NL)**

(56) References cited:
**US-A1- 2007 142 213   US-A1- 2010 065 476**
**US-A1- 2010 129 921   US-A1- 2010 159 609**
**US-A1- 2012 296 145   US-B1- 6 528 316**

• **JIA CUI ET AL: "Identification of acidic species in chloroaluminate ionic liquid catalysts", JOURNAL OF CATALYSIS., vol. 320, 11 October 2014 (2014-10-11), pages 26-32, XP055296287, US ISSN: 0021-9517, DOI: 10.1016/j.jcat.2014.09.004**

**Description**

Field of the invention

The present invention provides a process for monitoring the catalytic activity of and for the regeneration of the ionic liquid in continuous conversion of an olefin in an alkylation reaction and a process for preparing an alkylate using an ionic liquid of which the catalytic activity of the ionic liquid is determined using said monitoring process.

Background of the invention

Acidic ionic liquids (ILs), such as chloroalumininates, are successfully being used as environmentally friendly catalysts for the alkylation of 2-butene with isobutane or of benzene with an alphaolefin or an alkyl halide. The control of the catalytic activity and the regeneration of these ILs are important features for the industrial application. Catalytic activity is related to the acidity of these acidic ionic liquids. Therefore, interest in methods for monitoring the acidity of ionic liquids to enable and improve the control of the active species in ionic liquids has increased.

US2011/0184219 discloses a process to determine the ionic liquid catalyst deactivation by hydrolyzing a sample of ionic liquid catalyst, followed by titrating the hydrolyzed sample with a basic reagent to determine a volume of the basic reagent necessary to neutralize a Lewis acid species of the ionic liquid catalyst. The acid content from the sample in US2011/0184219 is then calculated from the volume of the used basic reagent.

WO2012/158259 discloses a method for monitoring an ionic liquid by contacting an infrared (IR) transmissive medium with the ionic liquid, followed by recording an IR spectrum of the ionic liquid, from which spectrum at least one chemical characteristic of the ionic liquid is quantified.

US 2010/0129921 A1 discloses a process for determining ionic liquid catalyst deactivation by determining the acidity of a hydrolysed sample of the used catalyst.

A problem of the processes disclosed in US2011/0184219 and WO2012/158259 is that said processes do not quantitatively characterize the activity of ionic liquids. In this way the level of catalytic activity of the ionic liquids cannot be monitored and consequently by lack of quantitative information on the extent of deactivation, the control of the regeneration in the process of continuous alkylation is difficult.

It is an object of the invention to provide a quantitative characterization method for the catalytic activity of acidic ionic liquids.

It is a further object of the present invention to monitor the catalytic activity level of acidic ionic liquids and to control the regeneration of the ionic liquid in the process of continuous alkylation by quantifying the amount of acid to be added.

One of the above or other objects may be achieved according to the present invention to provide a process for monitoring the catalytic activity of an ionic liquid and for the regeneration of the ionic liquid in continuous conversion of an olefin in an alkylation, comprising the steps of:

(a) providing a halogen-aluminate ionic liquid;
(b) subjecting a hydrocarbon mixture comprising at least an isoparaffin or an aromatic hydrocarbon and an olefin to a continuous alkylation reaction between the isoparaffin or the aromatic hydrocarbon and the olefin, wherein the hydrocarbon mixture is reacted with the ionic liquid of step (a) to obtain an effluent comprising at least an alkylate product and an ionic liquid phase;
(c) taking a sample of the ionic liquid phase;
(d) adding a portion of an organic compound to a sample of the ionic liquid phase of step (c) or adding a portion of the ionic liquid phase of step (c) to a sample of an organic compound;
(e) recording an infrared spectrum of a mixture as obtained in step (d) to obtain at least one absorption peak;
(f) repeating steps (d) and (e) until at least one absorption peak obtained in step (e) reaches a maximum value or a minimum value;
(g) determining at the maximum value or minimum value of the absorption peak of step (f): the total amount of the organic compound added in portions to the sample of the ionic liquid phase or determining the total amount of the ionic liquid phase added in portions to the sample of organic compound;
(h) calculating the catalytic activity of the ionic liquid on the basis of: the total amount of the organic compound added in portions as determined in step (g) or the total amount of ionic liquid phase added in portions as determined in step (g); and
(i) adding one or more aluminium halides or a mixture of aluminium halides to the continuous alkylation reaction of step (b) such that the activity of step (h) stays above the minimum level at which the conversion of the olefin in step (b) is lower than 100%.

It has now surprisingly been found according to the present invention that the catalytic activity of ionic liquids can be quantitatively characterized.

It is known that the catalytic activity of acidic chloroaluminate ionic liquids originates from the Lewis acid in chloroaluminate ionic liquids. The relationship between the catalytic activity of chloroaluminate ionic liquids and the Lewis acid $Al_2Cl_7^-$ in the chloroaluminate ionic liquids is described in for instance J. Cui, J. de With, P.A.A. Klusener, X.H. Su, X.H. Meng, R. Zhang, Z.C. Liu, C.M. Xu and H.Y. Liu, "Identification of acidic species in chloroaluminate ionic liquid catalysts", J. Catal., 320 (2014) 26.

By using in-situ infrared-complexometric titration the $Al_2Cl_7^-$ active component in the chloroaluminate ionic liquids during continuous alkylation can be quantitatively characterized.

In this way, the catalytic activity of the ionic liquid can be monitored with complexometric titration, by using infrared spectroscopy, preferably by using *in-situ* infrared spectroscopy.

Another advantage of the present invention is that by monitoring the catalytic activity of an ionic liquid, it can be determined at which activity the alkylation activity is too low for total conversion of the olefin. Therewith, the regeneration of the ionic liquid can be controlled in the process of continuous alkylation by adding a quantified amount of acid.

FT-IR is a method that can be applied easily and quickly in a plant laboratory with a small sample.

A further advantage is that the addition of fresh ionic liquid is avoided and that cheaper aluminium halides can be added to the process, which addition is also optimized by monitoring the catalytic activity.

In Figure 1 a FT-IR spectrum of the effect of the titration of an ionic liquid with acetone is shown.

In Figure 2 the determination of titration endpoints of the titration of the ionic liquid with acetone is shown.

In step (a) of the process according to the present invention a halogen-aluminate ionic liquid is provided. Processes to prepare halogen-aluminate ionic liquids are known in the art and are therefore not discussed here in detail. Preparation of halogen-aluminate ionic liquids is for example described in US7285698, WO2011/015639 and WO2015/028514.

Preferably, the halogen-aluminate ionic liquid is a chloroaluminate ionic liquid. The preparation of an acidic chloroaluminate ionic liquid has been described in e.g. WO2015/028514.

In step (b) of the process according to the present invention a hydrocarbon mixture comprising at least an isoparaffin or an aromatic hydrocarbon and an olefin is subjected to a continuous alkylation reaction between the isoparaffin or the aromatic hydrocarbon and the olefin, wherein the hydrocarbon mixture is reacted with the ionic liquid of step (a) to obtain an effluent comprising at least an alkylate product and an ionic liquid phase.

Continuous alkylation reactions between an isoparaffin or an aromatic hydrocarbon and an olefin are known in the art and therefore not described here in detail. These continuous alkylation reactions are for example described in US 7285698, WO2011/015639, WO2015/028514 and in US2010/0160703.

In step (c) of the process according to the present invention a sample of the ionic liquid phase of step b) is taken. Preferably, the effluent of step (b) is separated to obtain a hydrocarbon-rich phase and an ionic liquid phase.

Due to the low affinity of the ionic liquid for hydrocarbons and the difference in density between the hydrocarbons and the ionic liquid catalyst, the separation between the two phases is suitably done using for example well known settler means, wherein the hydrocarbons and catalyst separate into an upper predominantly hydrocarbon-rich phase and a lower predominantly catalyst (=ionic liquid phase) or by using any other suitable liquid/liquid separator. Such liquid/liquid separators are known to the skilled person and include cyclone and centrifugal separator.

In step (d) of the process according to the present invention a portion of an organic compound is added to a sample of the ionic liquid of step (c) or a portion of the ionic liquid of step (c) is added to a sample of an organic compound.

Suitably, the organic compound of step (d) contains a nitrogen group, oxygen group and/or sulphur group. In addition, the organic group which contains a nitrogen group, oxygen group and/or sulphur group is selected from the group consisting of alcohols, ketones, ethers, tetrahydrofurans, aldehydes, mercaptans, sulphur ethers, thiophenes, pyridines, nitro-aromates and derivatives thereof. More preferably, the organic group which contains a nitrogen group, oxygen group and/or sulphur group is selected from the group consisting of ethanol, acetone, diethyl ether, tetrahydrofuran, nitrobenzene, meta-methyl nitrobenzene, pyridine, and 2,6-dimethyl pyridine.

Most preferred organic group is nitrobenzene, acetone, tetrahydrofuran, ethanol, or diethyl ether.

In a first embodiment in step (d) of the process according to the present invention a portion of the organic compound is added to a sample of the ionic liquid to obtain a mixture.

In a second embodiment in step (d) of the process according to the present invention a portion of the ionic liquid is added to a sample of the organic compound to obtain a mixture.

Preferably, a sample of the ionic liquid is titrated with a portion of the organic compound or a sample of the organic compound is titrated with a portion of the ionic liquid. More preferably, the titration is complexometric titration. Titration, and in specific complexometric titration, is a technique known in the art and therefore not described here in detail.

Complexometric titration techniques are for example described in G. Schwarzenbach and H.A. Flasch, "Complexometric titrations", 2nd Ed., Methuen (1969).

This principle of the titration in this embodiments is related to monitoring the formation of a product between the organic compound and the acidic species in the ionic liquid and/or in case of adding ionic liquid for the organic compound also to the monitoring the disappearance of the organic compound. The monitoring in step (e) can be performed using spectroscopic techniques.

Suitably, the organic compound or the ionic liquid is used as a mixture using a solvent as diluent, preferred solvent is dichloromethane. Dichloromethane is the preferred solvent since said solvent does not react with the ionic liquid.

Preferably, the ionic liquid is used as a mixture using a solvent as diluent.

By using a solvent for the ionic liquid the advantage is that it lowers the viscosity and makes the mixing with the organic compound faster. So it fastens the reaction between acidic sites of the ionic liquid and organic compound and makes the titration more accurate.

The volume ratio of the solvent to the ionic liquid or the organic compound is preferably 0.5 to 20.

In step (e) of the process according to the present invention an infrared spectrum of the mixture as obtained in step (d) is recorded to obtain at least one absorption peak. Preferably, the infrared spectrum is recorded with a Fourier Transform Infrared Spectrometer (FT-IR). The use of FT-IR for following titration is a method known in the art and therefore not described here in detail. FT-IR for following titration is for example described in D. Li, J. Sedman, D.L. Garcia-Gonzalez, and F.R. van de Voort, "Automated Acid Content Determination in Lubricants by FTIR Spectroscopy as an Alternative to Acid Number Determination", Journal of ASTM International, Vol. 6, No. 6 (2009) Paper ID JAI102110.

Preferably, the infrared spectrum of steps (e) en (f) is recorded *in situ* during step (d), (e) and (f).

In the present invention by the term *"in situ"* is meant recording infrared spectra during titration.

The absorption peak in step (e) may result from the reaction product between the ionic liquid and the organic compound. In step (e) preferably one or more absorption peaks are obtained corresponding to one or more reaction products between the ionic liquid and the organic compound. Preferably, the absorption peak may result from the reaction product between the acidic chloroaluminate ionic liquid and the functional groups in the organic compounds containing nitrogen, oxygen and/or sulphur.

In alternative embodiments of this invention, in step (e) of the process according to the invention a Nuclear Magnetic Resonance (NMR) spectrum of a mixture as obtained in step (d) is recorded to obtain signals related to the reaction product of acidic ionic liquid and the organic compound and/or the disappearance of the organic compound. In other alternative embodiments of this invention other analytical techniques are used, such as ultra violet spectroscopy or colourimetry, that are sensitive to selectively monitor the formation of the reaction product of acidic ionic liquid and the organic compound and/or the disappearance of organic compound.

In the case that in step (d) a portion of the ionic liquid is added to a sample of the organic compound, the absorption peak in step (e) may result from the organic compound. Preferably, the absorption peak may result from the functional groups in the organic compounds containing nitrogen, oxygen or sulphur.

In step (f) of the process according to the present invention steps (d) and (e) are repeated until at least one absorption peak obtained in step (e) reaches a maximum value or a minimum value.

In the case that in step (d) a portion of the organic compound is added to a sample of the ionic liquid, at least one of the absorption peaks corresponding to one or more reaction products between the ionic liquid and the organic compound preferably reaches a maximum value in step (f).

In the case that in step (d) a portion of the ionic liquid is added to a sample of the organic compound, at least one absorption peak resulting from the organic compound reaches a minimum in step (f). As indicated above, the absorption peak may result from the functional groups in the organic compounds containing nitrogen, oxygen or sulphur.

In a first embodiment of the present invention, in step (d) a portion of the organic compound is added to a sample of ionic liquid to obtain a mixture. Preferably, of this mixture an infrared spectrum is recorded in step (e) to obtain a first absorption peak. In step (f) the first absorption peak corresponding to a first product between the ionic liquid and the organic compound reaches a maximum and at further repeating steps (d) en (e) a second absorption peak corresponding to a second product between the ionic liquid and organic compound reaches a maximum.

This second product may result from reaction between the first product and the functional groups in the organic compounds containing nitrogen, oxygen and/or sulphur. The first product may therefore be converted in the second product and may therefore disappear. Therefore, in step (f) at least one absorption peak corresponding to a product between the ionic liquid and the organic compound reaches a maximum and at further repeating steps (d) en (e) the same absorption peak reaches a minimum.

Preferably, depending on the type of organic compound used in step (d) and in step (e) at least one absorption peak corresponding to a product between the ionic liquid and the organic compound reaches a maximum and at further repeating steps (d) en (e) the same absorption peak reaches a minimum. Typically, some organic compound result in a second absorption peak.

In step (g) of the process according to the present invention, at the maximum value or minimum value of the absorption peak of step (f) the total amount of the organic compound added in portions to the sample of the ionic liquid is determined or the total amount of the ionic liquid added in portions to the sample of organic compound is determined.

In the first embodiment as indicated above, by addition of the organic compound in portions to the sample of the ionic liquid in step (f) at least one absorption peak corresponding to a product between the ionic liquid and the organic compound reaches a maximum and at further repeating steps (d) en (e) the same absorption peak reaches a minimum. Therefore, in step (g) of the first embodiment the total amounts of the organic compound added in portions to the sample of the ionic liquid is determined at which in step (f) one or more absorption peaks corresponding to a product between the ionic liquid and the organic compound reach a maximum or a minimum after first having reached a maximum.

In the second embodiment of the present invention, in step (d) a portion of the ionic liquid is added to a sample of the organic compound to obtain a mixture.

Typically, in the beginning of step (d) an infrared spectrum of only the organic compound is recorded because a reaction product between the ionic liquid and the organic compound may have not be formed.

Preferably, in step (e) at least one absorption peak is obtained corresponding to the organic compound. As more ionic liquid is added to a sample of organic compound a product may be obtained resulting from reaction between the acidic ionic liquid, preferably chloroaluminate ionic liquid, and the functional groups in the organic compounds containing nitrogen, oxygen and/or sulphur. The organic compound may therefore be converted in said product and may therefore disappear.

In the second embodiment as indicated above, by addition of the ionic liquid in portions to the sample of the organic compound, in step (f) a minimum is reached of the absorption peak corresponding to the organic compound and a maximum of the absorption peak corresponding to a product between the ionic liquid and the organic compound.

Therefore, in step (g) of the second embodiment the total amount of the ionic liquid added in portions to the sample of organic compound is determined at which in step (f) a minimum is reached of the absorption peak corresponding to the organic compound or a maximum of the absorption peak corresponding to the product between the ionic liquid and the organic compound.

In step (h) of the process according to the present invention the catalytic activity of the ionic liquid is calculated on the basis of: the total amount of the organic compound added in portions as determined in step (g) or the total amount of ionic liquid added in portions as determined in step (g).

The catalytic activity of the ionic liquid according to the present invention is defined as the ratio between the amount of organic compound and the amount of ionic liquid added at reaching the minimum or maximum as obtained in step (f).

In practice any unit for the ratio of amounts of organic compound and ionic liquid can be used to define an "activity index" as appropriate for the specific combination of organic compound and ionic liquid, such as: g indicator/100 g IL, mol indicator/mol IL, etc.

The catalytic activity may for instance be calculated with the formula as indicated below.

$$AI_{IL} = \frac{100 \cdot m_{IN}}{m_{IL} \cdot M_{IN}}$$

In the formula, $AI_{IL}$ is the "activity index" of the ionic liquid, $m_{IN}$ is the mass of the organic compound usage at the titration end point or the mass of the sample of organic compound in case ionic liquid was added to the organic compound, $M_{IN}$ is the molecular mass of the organic compound, and $m_{IL}$ is the mass of the sample of the ionic liquid or the mass of the amount of ionic liquid added to the organic compound sample at the titration end point.

In the first embodiment, in step (h) the catalytic activity of the ionic liquid is determined by the ratio of the total amount of the organic compound added in portions as determined in step (g) and the amount of the sample of ionic liquid of step (d).

In the second embodiment, in step (h) the catalytic activity of the ionic liquid is determined by the ratio of the amount of the sample of organic compound of step (d) and the total amount of ionic liquid added in portions as determined in step (g).

In step (i) of the process according to the present invention, one or more aluminium halides or a mixture of aluminium halides to the continuous alkylation reaction of step (b) is added such that the activity of step (h) stays above the minimum level at which the conversion is lower than 100%.

The continuous alkylation reaction is preferably controlled in such a way that the aluminium halide is added to the reaction of step (b) whenever the catalytic activity decreases to a level at which the level of activity is 10% higher (control level) than the level of activity at which the conversion is 100% (lower alarm level).

Therefore, in order to have enough time to add the aluminium halide to the reaction of step (b), the continuous alkylation reaction may be further controlled in such a way that the level of activity is always 20% higher (set value) than the level of activity at which the conversion is 100% (lower alarm level).

Typically, the lower alarm level, the control level and the set value level are higher than the minimum level in step (i) at which the conversion of the olefin in step (b) is lower than 100%.

In a further aspect the present invention provides a process to prepare an alkylate product, the process at least comprising the steps:

(aa) providing a hydrocarbon mixture comprising at least an isoparaffin or an aromatic hydrocarbon and an olefin;

(bb) subjecting the mixture of step (aa) to an alkylation reaction between the isoparaffin or the aromatic hydrocarbon and the olefin, wherein the hydrocarbon mixture is reacted with an ionic liquid to obtain an effluent comprising at least an alkylate product;

(cc) separating the effluent of step (bb), thereby obtaining a hydrocarbon-rich phase and an ionic liquid-rich phase;

(dd) fractionating the hydrocarbon-rich phase of step (cc), thereby obtaining at least the alkylate product and a isoparaffin-comprising stream or an aromatic hydrocarbon-comprising stream; and

(ee) recycling of the ionic liquid-rich phase of step (cc) to step (bb), wherein the catalytic activity of the ionic liquid of step (bb) and of the ionic liquid rich phase (cc) is determined with a process for monitoring the catalytic activity of an ionic liquid according to the present invention.

Process to prepare an alkylate product are known in the art and therefore not described here in detail. Process to prepare alkylate products comprising steps (aa) to (ee) are for example described in US 7285698, WO2011/015639, in WO2015/028514 and US20100160703, but the processes disclosed in the prior art, such as US 7285698, WO2011/015639, WO2015/028514 and in US20100160703 do not include determination of the catalytic activity of the ionic liquid of step (bb) and of the ionic liquid rich phase of step (cc) as determined with the process for monitoring the catalytic activity of an ionic liquid according the present invention The invention is illustrated by the following nonlimiting examples.

Example 1 Preparation of ionic liquid (IL)

Example 1.1 Preparation of ionic liquid $Et_3NHCl$-$2.0AlCl_3$ (IL-1)

$Et_3NHCl$ and $AlCl_3$ were obtained from Aladdin Industrial Inc.

137.7 g of $Et_3NHCl$ (1 mol) was placed in a 500 mL flask under $N_2$ atmosphere. Subsequently, 133.3 g of $AlCl_3$ (1 mol) was added into the flask. A reaction started and the mixture was stirred while the temperature rose to 100 °C by the exothermic reaction. The mixture was heated as soon as the temperature started to drop and kept at 120 °C for at least 2 hours by external heating. Then another portion of 133.3 g of $AlCl_3$ (1 mol) was added into the flask. The temperature of the mixture rose to 150 °C. The temperature of mixture was kept at 150 °C for at least 4 hours using external heating until a homogeneous liquid was obtained. The resulting liquid, being 404.3 g of ionic liquid IL-1, was allowed to cool down to room temperature.

Example 1.2 Preparation of ionic liquid $Et_3NHCl$-$2.0AlBr_3$ (IL-2)

$Et_3NHCl$ and $AlBr_3$ were obtained from Aladdin Industrial Inc.

137.7 g of $Et_3NHCl$ (1.0 mol) was placed in a 500 mL flask under $N_2$ atmosphere. Subsequently, 266.7 g of $AlBr_3$ (1.0 mol) was added into the flask. A reaction started and the mixture was stirred while the temperature rose to 100 °C by the exothermic reaction. The mixture was heated as soon as the temperature started to drop and kept at 120 °C for at least 2 hours by external heating. Then another portion of 266.7 g of $AlBr_3$ (1.0 mol) was added into the flask. The temperature of the mixture rose to 150 °C. The temperature of mixture was kept at 150 °C for at least 4 hours using external heating until a homogeneous liquid was obtained. The resulting liquid, being 671.1 g of ionic liquid IL-2, was allowed to cool down to room temperature.

Example 2 Alkylation tests

Example 2.1 Alkylation tests with IL-1 and Preparation of IL-1-deactived

200 g of IL-1 was placed into a 500 mL autoclave. The autoclave was closed, the stirrer was started, and the temperature inside the autoclave was controlled at 20 °C. C4 feed with an I/O ratio (isobutane/2-butene) of 10:1 (mol/mol) was pumped through a filter and a dryer, and then entered into the autoclave. The feed rate was controlled at 900 mL/h by the plunger pump. The pressure in the autoclave was maintained at 0.5 MPa to keep the reactants and product in liquid phase. During reaction and filling the autoclave, the reaction system was separating into two phases due to the differences in density. The upper part of the reaction mixture in the autoclave was the unreacted feed and products, while the lower part consisted of a mixture of composite ionic liquid and hydrocarbons. The upper part of the reaction mixture was collected via an overflow into a collection tank. Samples were taken from the overflow after certain amounts of feed fed into the autoclave to check for the conversion of 2-butene. After certain amounts of feed fed into the autoclave the feed and the stirrer were stopped and after 5 min a sample of the lower part, consisting mainly of composite ionic liquid, was taken from the bottom of the autoclave; at the same moment also a sample was taken from the overflow to check for the conversion of 2-butene (see Table 1), after which the stirring and the C4 feed was continued. The samples taken from the bottom of the autoclave were decompressed to remove dissolved hydrocarbon and were subsequently centrifuged to remove solid formed during reaction. After 28.9 kg of feed fed into the autoclave the conversion of 2-butene had been lower than 90 % (75 %). Then the feed and the stirrer were stopped and after 30 min a sample of the lower part, consisting mainly of ionic liquid, was taken from the bottom of the autoclave (IL-1-deactived); at the same moment also a sample was taken from the overflow to check for the conversion of 2-butene (41 %).

Example 2.2 Alkylation tests with IL-2 and Preparation of IL-2-deactived

The procedure of example 2.1 was repeated with 300 g of composite IL-2. After 25.7 kg of feed fed into the autoclave the conversion of 2-butene had been lower than 90 % (81 %). Then the feed and the stirrer were stopped and after 30 min a sample of the lower part, consisting mainly of ionic liquid, was taken from the bottom of the autoclave (IL-2-deactived); at the same moment also a sample was taken from the overflow to check for the conversion of 2-butene (48 %) (Table 1).

Example 3 Regeneration tests

Example 3.1 Preparation of IL-1-regenerated

After alkylation test of example 2.1, the hydrocarbon phase was removed from the autoclave, and the 66.7 g of $AlCl_3$ (0.5 mol) was added into the autoclave. The mixture was stirred and heated to 80 °C at least 4 hours until a homogeneous liquid was obtained. The resulting liquid, being IL-1-regenerated, was allowed to cool down to room temperature.

Example 3.2 Preparation of IL-2-regenerated

After alkylation test of example 2.2, the hydrocarbon phase was removed from the autoclave, and the 133.3 g of $AlBr_3$ (0.5 mol) was added into the autoclave. The mixture was stirred and heated to 80 °C at least 4 hours until a homogeneous liquid was obtained. The resulting liquid, being IL-2-regenerated, was allowed to cool down to room temperature.

Example 4 Alkylation tests with regenerated IL

Example 4.1 Alkylation tests with IL-1-regenerated

After procedure of example 3.1, the autoclave was closed, and the procedure of example 2.1 (Alkylation tests) was repeated with IL-1-regenerated. After 23.1 kg of feed fed into the autoclave the conversion of 2-butene had been lower than 90 % (68 %). Then the feed and the stirrer were stopped and after 30 min a sample of the lower part, consisting mainly of ionic liquid, was taken from the bottom of the autoclave; at the same moment also a sample was taken from the overflow to check for the conversion of 2-butene (35 %) (see Table 1).

Example 4.2 Alkylation tests with IL-2-regenerated

After procedure of example 3.2, the autoclave was closed, and the procedure of example 2.2 (Alkylation tests) was repeated with IL-2-regenerated. After 18.5 kg of feed fed into the autoclave the conversion of 2-butene had been lower than 90 % (85 %). Then the feed and the stirrer were stopped and after 30 min a sample of the lower part, consisting mainly of ionic liquid, was taken from the bottom of the autoclave; at the same moment also a sample was taken from the overflow to check for the conversion of 2-butene (50 %) (see Table 1).

Example 5 Determination of catalytic activity of IL with infrared spectroscopy

Example 5.1 Determination of catalytic activity of IL-1 with infrared spectroscopy by titration of IL-1 with acetone.

IL-1 (20.012) g was placed in a 50 mL flask under $N_2$ atmosphere and was stirred continuously during the titration. The titration was performed by addition of acetone (supplied by Aladdin Company) in portions while FT-IR spectra of the mixture were recorded *in situ* by an infrared detection apparatus at equal time intervals.

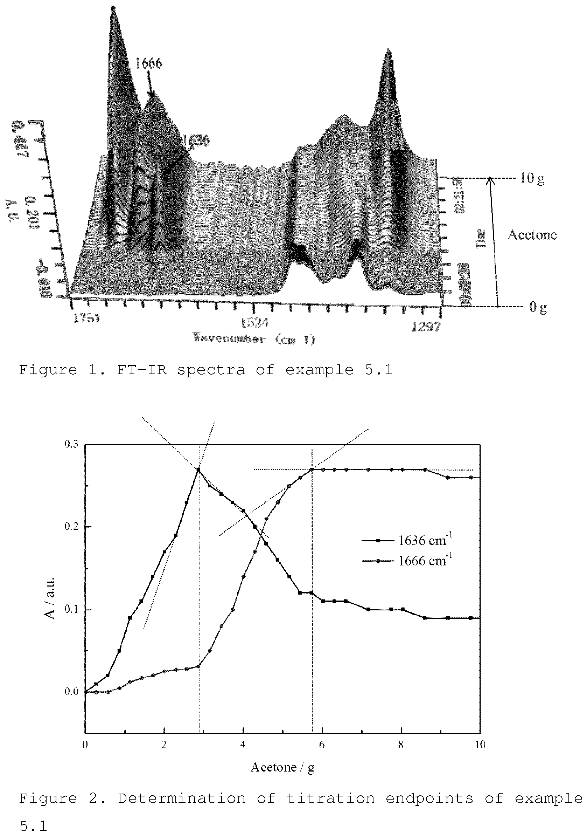

Figure 1 shows that during titration initially an absorption peak at 1666 cm$^{-1}$ appeared and when it reached its maximum another peak at 1636 cm$^{-1}$ appeared, while acetone was added in portions. The intensity changes of these two peaks were tracked by the *in-situ* infrared apparatus and plotted against the amount of acetone added (Fig. 2). The titration end points were determined at the moment that the intensity of the 1636 cm$^{-1}$ peak reached its maximum and when the intensity of the 1666 cm$^{-1}$ was not increasing anymore upon the addition of acetone. The acetone usage was 2.861 g at the first titration end point and 5.7 g at the second titration end point. The second titration end point is related to the interaction of two molar equivalents of acetone with the catalyst; so the acetone usage at this second titration end point needs to be divided by 2, to be used in the calculation of the catalytic activity. The catalytic activity of IL-1 ionic liquid defined as "activity index" was 0.246 mol indicator/100 g of IL (see Table 1).

Example 5.2 -5.10 Determination of catalytic activity of IL of examples 1.1 to 4.2 with infrared spectroscopy by titration of IL with acetone.

The procedure of example 5.1 was repeated for determining the catalytic activity of IL-1-deactivated, IL-1-regenerated, IL-2, IL-2-deactivated and IL-2-regenerated. The results are shown in Table 1.

Table 1 Catalytic activity of IL defined as "activity index" as determined with infrared spectroscopy in examples 5.1 - 5.10

| Alkylation example (Titration example) | Titre (titration example) | C4 feed fed to autoclave (kg) | Olefin conversion (%) | "Activity index" (mol acetone/ 100 g CIL) |
|---|---|---|---|---|
| 1.1 | IL-1 (5.1) | 0 | - | 0.247 |
| 2.1 | IL-1 during alkylation test (5.2) | 10.0 | 100 | 0.152 |
|  |  | 20.0 | 100 | 0.075 |
|  |  | *28.9 | 75 |  |
|  | IL-1-deactived (5.3) | 39.2 | 41 | 0.020 |
| 3.1 | IL-1-regenerated (5.4) | 0 | - | 0.177 |
| 4.1 | IL-1-regenerated during alkylation test (5.5) | 10.0 | 100 | 0.098 |
|  |  | 20.0 | 100 | 0.041 |
|  |  | *23.1 | 68 |  |
|  |  | 23.4 | 35 | 0.016 |
| 2.2 | IL-2 (5.6) | 0 | - | 0.150 |
| 2.2 | IL-2 during alkylation test (5.7) | 20.0 | 100 | 0.047 |
|  |  | *25.7 | 81 |  |
|  | IL-2-deactived (5.8) | 26.0 | 48 | 0.011 |
| 3.2 | IL-2-regenerated (5.9) | 0 | - | 0.110 |
| 4.2 | IL-2-regenerated during alkylation test (5.10) | 10.0 | 100 | 0.08 |
|  |  | *18.5 | 85 |  |
|  |  | 18.8 | 50 | 0.011 |
| * only sample of overflow was taken. | | | | |

Discussion

The "activity indices" as determined in examples 5.1-5.10 are summarized in Table 1 showing that the catalytic activity of acidic ionic liquids can be monitored with infrared spectroscopy. Further, Table 1 shows that with infrared spectroscopy it was determined at which activity the alkylation activity was too low for the total conversion of the olefin (IL-1 deactivated (example 5.3) and IL-2 deactivated (example 5.8). Therewith, the catalytic activities of IL-1 deactivated and IL-2 deactivated were increased upon the addition of $AlCl_3$ (example 5.5) and $AlBr_3$ (example 5.10). This indicates that a high amount of Lewis acidity, determined by the amount of $AlCl_3$ and $AlBr_3$, may influence the catalytic activity (activity index) in a positive manner.

Examples 5.2, 5.5, 5.7 and 5.10 show that the activity index can be monitored by sampling ionic liquid from the continuous alkylation process. The results in Table 1 shows the activity index, being a measure of the Lewis acidity, decreased gradually. This indicates that deactivated ionic liquid has little, but insufficient Lewis activity to completely convert the olefin in the alkylation reaction. By using the method according to the present invention it can be determined at which activity index the alkylation activity is too low for total conversion of the olefin and at which activity level $AlCl_3$ or $AlBr_3$ may be added.

**Claims**

1. Process for monitoring the catalytic activity of an ionic liquid and for the regeneration of the ionic liquid in continuous conversion of an olefin in an alkylation, comprising the steps of:

   (a) providing a halogen-aluminate ionic liquid;
   (b) subjecting a hydrocarbon mixture comprising at least an isoparaffin or an aromatic hydrocarbon and an olefin to a continuous alkylation reaction between the isoparaffin or the aromatic hydrocarbon and the olefin, wherein the hydrocarbon mixture is reacted with the ionic liquid of step (a) to obtain an effluent comprising at least an alkylate product and an ionic liquid phase;
   (c) taking a sample of the ionic liquid phase;
   (d) adding a portion of an organic compound to a sample of the ionic liquid phase of step (c) or adding a portion of the ionic liquid phase of step (c) to a sample of an organic compound;
   (e) recording an infrared spectrum of a mixture as obtained in step (d) to obtain at least one absorption peak, wherein the adsorption peak relates to a product between the organic compound and an acidic species in the ionic liquid or to the disappearance of the organic compound when adding ionic liquid to the organic compound;
   (f) repeating steps (d) and (e) until at least one absorption peak obtained in step (e) reaches a maximum value or a minimum value;
   (g) determining at the maximum value or minimum value of the absorption peak of step (f): the total amount of the organic compound added in portions to the sample of the ionic liquid phase or determining the total amount of the ionic liquid phase added in portions to the sample of organic compound;
   (h) calculating the catalytic activity of the ionic liquid on the basis of: the total amount of the organic compound added in portions as determined in step (g) or the total amount of ionic liquid phase added in portions as determined in step (g); and
   (i) adding one or more aluminium halides or a mixture of aluminium halides to the continuous alkylation reaction of step (b) when the activity of step (h) decreases to a level of activity 10% higher than the level of activity at which the conversion of the olefin in step (b) is 100%.

2. Process according to claim 1, wherein the infrared spectrum of steps (e) and (f) is recorded *in situ* during step (d), (e) and (f).

3. Process according to claim 1 or 2, wherein in step (e) one or more absorption peaks are obtained corresponding to one or more products between the ionic liquid phase and the organic compound.

4. Process according to claim 3, wherein in step (f) at least one absorption peak corresponding to a product between the ionic liquid phase and the organic compound reaches a maximum.

5. Process according to any one of claims 1 to 4, wherein in step (d) a portion of the organic compound is added to a sample of ionic liquid phase.

6. Process according to claim 5, wherein in step (f) a first absorption peak corresponding to a first product between the ionic liquid phase and the organic compound reaches a maximum and at further repeating steps (d) and (e) a second absorption peak corresponding to a second product between the ionic liquid phase and the organic compound reaches a maximum.

7. Process according to any one of claims 5 or 6, wherein in step (f) at least one absorption peak corresponding to a product between the ionic liquid phase and the organic compound reaches a maximum and at further repeating steps (d) and (e) the same absorption peak reaches a minimum.

8. Process according to any one of claims 4 to 7, wherein in step (g) the total amounts of the organic compound added in portions to the sample of the ionic liquid phase is determined at which in step (f) one or more absorption peaks corresponding to a product between the ionic liquid phase and the organic compound reach a maximum or a minimum after first having reached a maximum.

9. Process according to any one of claims 1 to 4, wherein in step (d) a portion of the ionic liquid phase is added to a sample of the organic compound.

10. Process according to claim 9, wherein in step (e) at least one absorption peak is obtained corresponding to the organic compound.

11. Process according to claims 9 and 10, wherein in step (f) the absorption peak corresponding to the organic compound reaches a minimum.

12. Process according to any one of claims 1 to 4 and 9 to 11, wherein in step (g) the total amount of the ionic liquid phase added in portions to the sample of organic compound is determined at which in step (f) a minimum is reached of the absorption peak corresponding to the organic compound or a maximum of the absorption peak corresponding to the product between the ionic liquid phase and the organic compound.

13. Process according to any one of claims 1 to 8, wherein in step (h) the catalytic activity of the ionic liquid phase is determined by the ratio of the total amount of the organic compound added in portions as determined in step (g) and the amount of the sample of ionic liquid phase of step (d).

14. Process according to any one of claims 1 to 4 and 9 to 12, wherein in step (h) the catalytic activity of the ionic liquid is determined by the ratio of the total amount of the sample of organic compound of step (d) and the total amount of ionic liquid phase added in portions as determined in step (g).

15. Process to prepare an alkylate product, the process at least comprising the steps:

(aa) providing a hydrocarbon mixture comprising at least an isoparaffin and an olefin;
(bb) subjecting the mixture of step (aa) to an alkylation reaction between the isoparaffin and the olefin, wherein the hydrocarbon mixture is reacted with an ionic liquid to obtain an effluent comprising at least an alkylate product;
(cc) separating the effluent of step (bb), thereby obtaining a hydrocarbon-rich phase and an ionic liquid-rich phase;
(dd) fractionating the hydrocarbon-rich phase of step (cc), thereby obtaining at least the alkylate product and a isoparaffin-comprising stream; and
(ee) recycling of the ionic liquid-rich phase of step (cc) to step (bb), wherein the catalytic activity of the ionic liquid of step (bb) and of the ionic liquid rich phase of step (cc) is determined according to any one of claims 1 to 14.

**Patentansprüche**

1. Vorgang zum Überwachen der katalytischen Aktivität einer ionischen Flüssigkeit und zum Aufbereiten der ionischen Flüssigkeit in einem Dauerumwandeln eines Olefins in einer Alkylierung, der die folgenden Schritte umfasst:

(a) Bereitstellen einer ionischen Halogenaluminat-Flüssigkeit;
(b) Unterziehen einer Kohlenwasserstoffmischung, die wenigstens ein Isoparaffin oder einen aromatischen Kohlenwasserstoff und ein Olefin umfasst, einer Daueralkylierungsreaktion zwischen dem Isoparaffin oder dem aromatischen Kohlenwasserstoff und dem Olefin, wobei die Kohlenwasserstoffmischung mit der ionischen Flüssigkeit aus Schritt (a) umgesetzt wird, um ein Effluent zu gewinnen, das wenigstens ein Alkylatprodukt und eine ionische Flüssigphase umfasst;
(c) Entnehmen einer Probe der ionischen Flüssigphase;
(d) Hinzufügen eines Teils einer organischen Verbindung zu einer Probe der ionischen Flüssigphase aus Schritt (c) oder Hinzufügen eines Teils der ionischen Flüssigphase aus Schritt (c) zu einer Probe einer organischen Verbindung;
(e) Aufzeichnen eines Infrarotspektrums einer Mischung, wie in Schritt (d) gewonnen, um wenigstens eine Absorptionsspitze zu gewinnen, wobei sich die Adsorptionsspitze auf ein Produkt zwischen der organischen Verbindung und einer Säurespezies in der ionischen Flüssigkeit oder auf das Verschwinden der organischen Verbindung bezieht, wenn der organischen Verbindung eine ionische Flüssigkeit hinzugefügt wird;

(f) Wiederholen der Schritte (d) und (e), bis wenigstens eine in Schritt (e) gewonnener Absorptionsspitze einen Maximalwert oder einen Minimalwert erreicht;

(g) Bestimmenan dem Maximalwert und dem Minimalwert der Absorptionsspitze aus Schritt (f): der Gesamtmenge der inTeilen zu der Probe der ionischen Flüssigphase hinzugefügten organischen Verbindung oder Bestimmen der Gesamtmenge der in Teilen zu der Probe der organischen Verbindung hinzugefügten ionischen Flüssigphase;

(h) Berechnen der katalytischen Aktivität der ionischen Flüssigkeit auf der Grundlage von: der Gesamtmenge der in Teilen hinzugefügten organischen Verbindung, wie in Schritt (g) bestimmt, oder der Gesamtmenge der in Teilen hinzugefügten ionischen Flüssigphase, wie in Schritt (g) bestimmt; und

(i) Hinzufügen eines oder mehrerer Aluminiumhalogenide oder einer Mischung von Aluminiumhalogeniden zu der Daueralkylierungsreaktion aus Schritt (b), wenn die Aktivität aus Schritt (h) auf ein Aktivitätsniveau absinkt, das 10 % höher ist als das Aktivitätsniveau, auf dem die Umwandlung des Olefins in Schritt (b) 100 % beträgt.

2. Vorgang nach Anspruch 1, wobei das Infrarotspektrum der Schritte (e) und (f) in situ während des Schritts (d), (e) und (f) aufgezeichnet wird.

3. Vorgang nach Anspruch 1 oder 2, wobei in Schritt (e) eine oder mehrere Absorptionsspitzen gewonnen werden, die einem oder mehreren Produkten zwischen der ionischen Flüssigphase und der organischen Verbindung entsprechen.

4. Vorgang nach Anspruch 3, wobei in Schritt (f) wenigstens eine Absorptionsspitze, die einem Produkt zwischen der ionischen Flüssigphase und der organischen Verbindung entspricht, ein Maximum erreicht.

5. Vorgang nach einem der Ansprüche 1 bis 4, wobei in Schritt (d) ein Teil der organischen Verbindung zu einer Probe einer ionischen flüssigen Phase hinzugefügt wird.

6. Vorgang nach Anspruch 5, wobei in Schritt (f) eine erster Absorptionsspitze, die einem ersten Produkt zwischen der ionischen Flüssigphase und der organischen Verbindung entspricht, ein Maximum erreicht und bei weiteren Wiederholungsschritten (d) und (e) eine zweiter Absorptionsspitze, die einem zweiten Produkt zwischen ionischen Flüssigphase und der organischen Verbindung entspricht, ein Maximum erreicht.

7. Vorgang nach einem der Ansprüche 5 oder 6, wobei in Schritt (f) wenigstens eine Absorptionsspitze, die einem Produkt zwischen der ionischen Flüssigphase und der organischen Verbindung entspricht, ein Maximum erreicht und bei weiteren Wiederholungsschritten (d) und (e) die gleiche Absorptionsspitze ein Minimum erreicht.

8. Vorgang nach einem der Ansprüche 4 bis 7, wobei in Schritt (g) die Gesamtmengen der in Teilen zu der ionischen Flüssigphase hinzugefügten organischen Verbindung bestimmt werden, bei der in Schritt (f) eine oder mehrere Absorptionsspitzen, die einem Produkt zwischen der ionischen Flüssigphase und der organischen Verbindung entsprechen, ein Maximum oder ein Minimum nach dem vorherigen Erreichen eines Maximums erreichen.

9. Vorgang nach einem der Ansprüche 1 bis 4, wobei in Schritt (d) ein Teil der ionischen Flüssigphase zu einer Probe der organischen Verbindung hinzugefügt wird.

10. Vorgang nach Anspruch 9, wobei in Schritt (e) wenigstens eine Absorptionsspitze gewonnen wird, die der organischen Verbindung entspricht.

11. Vorgang nach den Ansprüchen 9 und 10, wobei in Schritt (f) die Absorptionsspitze, die der organischen Verbindung entspricht, ein Minimum erreicht.

12. Vorgang nach einem der Ansprüche 1 bis 4 und 9 bis 11, wobei in Schritt (g) die Gesamtmenge der in Teilen zu der Probe der organischen Verbindung hinzugefügten ionischen Flüssigphase bestimmt wird, bei der in Schritt (f) ein Minimum der Absorptionsspitze, die der organischen Verbindung entspricht, oder ein Maximum der Absorptionsspitze, die dem Produkt zwischen der ionischen Flüssigphase und der organischen Verbindung entspricht, erreicht wird.

13. Vorgang nach einem der Ansprüche 1 bis 8, wobei in Schritt (h) die katalytische Aktivität der ionischen Flüssigphase durch das Verhältnis der Gesamtmenge der in Teilen hinzugefügten organischen Verbindung, wie in Schritt (g) bestimmt, und der Menge der Probe der ionischen Flüssigphase aus Schritt (d) bestimmt wird.

**14.** Vorgang nach einem der Ansprüche 1 bis 4 und 9 bis 12, wobei in Schritt (h) die katalytische Aktivität der ionischen Flüssigkeit durch das Verhältnis der Gesamtmenge der Probe der organischen Verbindung aus Schritt (d) und der Gesamtmenge der in Teilen hinzugefügten ionischen Flüssigphase bestimmt wird, wie in Schritt (g) bestimmt.

**15.** Vorgang zum Herstellen eines Alkylatprodukts, wobei der Vorgang wenigstens die folgenden Schritte umfasst:

(aa) Bereitstellen einer Kohlenwasserstoffmischung, die wenigstens ein Isoparaffin und ein Olefin umfasst;
(bb) Unterziehen der Mischung aus Schritt (aa) einer Alkylierungsreaktion zwischen dem Isoparaffin und dem Olefin, wobei die Kohlenwasserstoffmischung mit einer ionischen Flüssigkeitumgesetzt wird, um ein Effluent zu gewinnen, das wenigstens ein Alkylatprodukt umfasst;
(cc) Abtrennen des Effluents aus Schritt (bb), wobei dadurch eine kohlenwasserstoffreiche Phase und eine ionische flüssigkeitsreiche Phase gewonnen werden;
(dd) Fraktionieren der kohlenwasserstoffreichen Phase aus Schritt (cc), wobei dadurch wenigstens das Alkylatprodukt und ein Isoparaffin umfassender Strom gewonnen werden; und
(ee) Wiederverwerten der ionischen flüssigkeitsreichen Phase aus Schritt (cc) bis Schritt (bb), wobei die katalytische Aktivität der ionischen Flüssigkeit aus Schritt (bb) und der ionischen flüssigkeitsreichen Phase aus Schritt (cc) nach einemder Ansprüche 1 bis 14 bestimmt wird.

**Revendications**

**1.** Procédé de surveillance de l'activité catalytique d'un liquide ionique et de régénération du liquide ionique lors de la conversion continue d'une oléfine pendant une alkylation, comprenant les étapes consistant à :

(a) fournir un liquide ionique halogène-aluminate ;
(b) soumettre un mélange d'hydrocarbures comprenant au moins une isoparaffine ou un hydrocarbure aromatique et une oléfine à une réaction d'alkylation continue entre l'isoparaffine ou l'hydrocarbure aromatique et l'oléfine, le mélange d'hydrocarbures étant mis en réaction au moyen du liquide ionique de l'étape (a) afin obtenir un effluent comprenant au moins un produit alkylate et une phase liquide ionique ;
(c) prélever un échantillon de la phase liquide ionique ;
(d) ajouter une portion d'un composé organique à un échantillon de la phase liquide ionique de l'étape (c) ou ajouter une portion de la phase liquide ionique de l'étape (c) à un échantillon d'un composé organique ;
(e) enregistrer un spectre infrarouge d'un mélange tel qu'obtenu à l'étape (d) afin d'obtenir au moins un pic d'absorption, le pic d'adsorption concernant un produit entre le composé organique et une espèce acide dans le liquide ionique ou la disparition du composé organique lors de l'ajout d'un liquide ionique au composé organique ;
(f) répéter les étapes (d) et (e) jusqu'à ce qu'au moins un pic d'absorption obtenu à l'étape (e) atteigne une valeur maximale ou une valeur minimale ;
(g) déterminer, à la valeur maximale ou à la valeur minimale du pic d'absorption de l'étape (f) : la quantité totale du composé organique ajouté en portions à l'échantillon de la phase liquide ionique ou déterminer la quantité totale de la phase liquide ionique ajoutée en portions à l'échantillon de composé organique ;
(h) calculer l'activité catalytique du liquide ionique en se basant sur : la quantité totale du composé organique ajoutée en portions telle que déterminée à l'étape (g) ou la quantité totale de la phase liquide ionique ajoutée en portions telle que déterminée à l'étape (g) ; et
(i) ajouter un ou plusieurs halogénures d'aluminium ou un mélange d'halogénures d'aluminium à la réaction d'alkylation continue de l'étape (b) lorsque l'activité de l'étape (h) diminue jusqu'à un niveau d'activité supérieur de 10 % par rapport au niveau d'activité auquel la conversion de l'oléfine à l'étape (b) est de 100 %.

**2.** Procédé selon la revendication 1, dans lequel le spectre infrarouge des étapes (e) et (f) est enregistré *in situ* au cours de l'étape (d), (e) et (f) .

**3.** Procédé selon la revendication 1 ou 2, dans lequel, à l'étape (e), un ou plusieurs pics d'absorption sont obtenus correspondant à un ou plusieurs produits entre la phase liquide ionique et le composé organique.

**4.** Procédé selon la revendication 3, dans lequel, à l'étape (f), au moins un pic d'absorption correspondant à un produit entre la phase liquide ionique et le composé organique atteint un maximum.

**5.** Procédé selon l'une quelconque des revendications 1 à 4, dans lequel, à l'étape (d), une portion du composé organique est ajoutée à un échantillon de la phase liquide ionique.

6. Procédé selon la revendication 5, dans lequel, à l'étape (f), un premier pic d'absorption correspondant à un premier produit entre la phase liquide ionique et le composé organique atteint un maximum et, lors d'autres étapes (d) et (e) se répétant, un second pic d'absorption correspondant à un second produit entre la phase liquide ionique et le composé organique atteint un maximum.

7. Procédé selon l'une quelconque des revendications 5 ou 6, dans lequel, à l'étape (f), au moins un pic d'absorption correspondant à un produit entre la phase liquide ionique et le composé organique atteint un maximum et, lors d'autres étapes (d) et (e) se répétant, le même pic d'absorption atteint un minimum.

8. Procédé selon l'une quelconque des revendications 4 à 7, dans lequel, à l'étape (g), les quantités totales du composé organique ajoutées en portions à l'échantillon de la phase liquide ionique sont déterminées à l'étape (f), selon laquelle un ou plusieurs pics d'absorption correspondant à un produit entre la phase liquide ionique et le composé organique atteignent un maximum ou un minimum après avoir tout d'abord atteint un maximum.

9. Procédé selon l'une quelconque des revendications 1 à 4, dans lequel, à l'étape (d), une portion de la phase liquide ionique est ajoutée à un échantillon du composé organique.

10. Procédé selon la revendication 9, dans lequel, à l'étape (e), au moins un pic d'absorption est obtenu correspondant au composé organique.

11. Procédé selon la revendication 9 et 10, dans lequel, à l'étape (f), le pic d'absorption correspondant au composé organique atteint un minimum.

12. Procédé selon l'une quelconque des revendications 1 à 4 et 9 à 11, dans lequel, à l'étape (g), la quantité totale de la phase liquide ionique ajoutée en portions à l'échantillon de composé organique est déterminée à l'étape (f), selon laquelle un minimum du pic d'absorption est atteint correspondant au composé organique ou un maximum du pic d'absorption est atteint correspondant au produit entre la phase liquide ionique et le composé organique.

13. Procédé selon l'une quelconque des revendications 1 à 8, dans lequel à l'étape (h) l'activité catalytique de la phase liquide ionique est déterminée par le rapport de la quantité totale du composé organique ajoutée en portions telle que déterminée à l'étape (g) et la quantité de l'échantillon de la phase liquide ionique de l'étape (d) .

14. Procédé selon l'une quelconque des revendications 1 à 4 et 9 à 12, dans lequel, à l'étape (h), l'activité catalytique du liquide ionique est déterminée par le rapport de la quantité totale de l'échantillon de composé organique de l'étape (d) et de la quantité totale de la phase liquide ionique ajoutée en portions telle que déterminée à l'étape (g).

15. Procédé de préparation d'un produit alkylate, le procédé comprenant au moins les étapes consistant à :

(aa) fournir un mélange d'hydrocarbures comprenant au moins une isoparaffine et une oléfine ;
(bb) soumettre le mélange de l'étape (aa) à une réaction d'alkylation entre l'isoparaffine et l'oléfine, le mélange d'hydrocarbures étant mis en réaction au moyen d'un liquide ionique afin d'obtenir un effluent comprenant au moins un produit alkylate ;
(cc) séparer l'effluent de l'étape (bb), afin d'obtenir une phase riche en hydrocarbures et une phase riche en liquide ionique ;
(dd) fractionner la phase riche en hydrocarbures de l'étape (cc), afin d'obtenir au moins le produit alkylate et un courant comprenant de l'isoparaffine ; et
(ee) recycler la phase riche en liquide ionique de l'étape (cc) à l'étape (bb), l'activité catalytique du liquide ionique de l'étape (bb) et de la phase riche en liquide ionique de l'étape (cc) étant déterminée selon l'une quelconque des revendications 1 à 14.

Figure 1. FT-IR spectra of example 5.1

Figure 2. Determination of titration endpoints of example 5.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20110184219 A
- WO 2012158259 A
- US 20100129921 A1
- US 7285698 B
- WO 2011015639 A
- WO 2015028514 A
- US 20100160703 A

### Non-patent literature cited in the description

- **J. CUI ; J. DE WITH ; P.A.A. KLUSENER ; X.H. SU ; X.H. MENG ; R. ZHANG ; Z.C. LIU ; C.M. XU ; H.Y. LIU.** Identification of acidic species in chloroaluminate ionic liquid catalysts. *J. Catal.,* 2014, vol. 320, 26
- **G. SCHWARZENBACH ; H.A. FLASCH.** *Complexometric titrations,* 1969
- **D. LI, J. SEDMAN ; D.L. GARCIA-GONZALEZ ; F.R. VAN DE VOORT.** Automated Acid Content Determination in Lubricants by FTIR Spectroscopy as an Alternative to Acid Number Determination. *Journal of ASTM International,* 2009, vol. 6 (6